(19) Europäisches Patentamt
European Patent Office
Office européen des brevets

(11) Veröffentlichungsnummer : **0 081 771**
**B1**

(12) # EUROPÄISCHE PATENTSCHRIFT

(45) Veröffentlichungstag der Patentschrift : **28.05.86**

(21) Anmeldenummer : **82111200.0**

(22) Anmeldetag : **03.12.82**

(51) Int. Cl.⁴ : **C 07 D495/04**, A 61 K 7/06, A 61 K 7/48 // (C07D495/04, 333:00, 235:00)

(54) **Heterocyclische Verbindungen, Herstellung dieser Verbindungen, kosmetische Mittel, die diese Verbindungen als Wirkstoffe enthalten, sowie Verwendung dieser Mittel zur Pflege des Haares oder der Haut.**

(30) Priorität : **10.12.81 CH 7897/81**

(43) Veröffentlichungstag der Anmeldung : **22.06.83 Patentblatt 83/25**

(45) Bekanntmachung des Hinweises auf die Patenterteilung : **28.05.86 Patentblatt 86/22**

(84) Benannte Vertragsstaaten : **AT BE CH DE FR GB IT LI NL SE**

(56) Entgegenhaltungen :
**FR-A- 1 588 770**
**US-A- 2 715 634**
**CHEMICAL ABSTRACTS, Band 80, Nr. 14, 1974, Seite 275, Nr. 100112h, Columbus, Ohio, USA**
**CHEMICAL ABSTRACTS, Band 77, Nr. 24, 11. Dezember 1972, Seite 281, Nr. 156274t, Columbus, Ohio, USA E. SHERR-BRUZER: "Additives which enhance the efficiency of cosmetics"**
**Moderne Arzneimittel-Wissensch. Verlagsgesel. mbH Stuttgart 5. Auflage - Seite 1460 (1980)**

(73) Patentinhaber : **Richardson-Vicks, Inc.**
**Ten Westport Road**
**Wilton, CT 06897 (US)**

(72) Erfinder : **Hostettler, Hans Ulrich, Dr.**
**Weidenhofweg 28**
**CH-4144 Arlesheim (CH)**
Erfinder : **Poncioni, Bruno, Dr.**
**Unterwartweg 29**
**CH-4132 Muttenz (CH)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 081 771 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Förderung der Fett- und Cholesterinsynthese der Haut sowie die Förderung des Haarwuchses durch Biotin (D-cis-Hexahydro-2-oxothieno [3,4-d] imidazol-4-valeriansäure) bei dessen örtlicher Anwendung ist bekannt. Diese Befunde, insbesondere der zweitgenannte, haben das Interesse der Kosmetik-Industrie erweckt. Es hat sich allerdings erwiesen, dass die Aufnahme von Biotin durch die menschliche Haut zu gering ist, um die gewünschten Wirkungen zufriedenstellend zu erzielen.

Es wurde nun überraschenderweise gefunden, dass gewisse Ester von Biotin, nämlich die $C_{2-4}$-Alkylester von Biotin, bei äusserlicher Anwendung durch die Haut besser aufgenommen werden als Biotin selbst, jedoch während oder nach der Aufnahme unter Einwirkung des menschlichen Organismus in Biotin umgewandelt werden.

Die vorliegende Erfindung betrifft somit die $C_{2-4}$-Alkylester von Biotin, d. h. Verbindungen der allgemeinen Formel

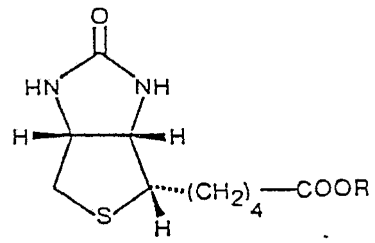

(I)

worin R $C_{2-4}$-Alkyl bedeutet, zur Anwendung als Wirkstoffe von Haarpflege- und Hautpflegemitteln.

Die Erfindung betrifft ferner Verbindungen der Formel I enthaltende kosmetische Haarpflege- und Hautpflegemittel.

Der oben verwendete Ausdruck « Alkyl » umfasst sowohl geradkettige als auch verzweigte Alkylgruppen. Somit umfasst « $C_{2-4}$-Alkyl » Aethyl, n-Propyl, Isopropyl, n-Butyl, Isobutyl, sek.Butyl und tert.Butyl. Die bevorzugte Alkylgruppe ist Aethyl, so dass eine besonders bevorzugte Verbindung der Formel I der Biotinäthylester ist.

Alkylester von Biotin werden in der US-A 2 715 634 beschrieben, eine therapeutische Aktivität dieser Substanzen wird jedoch nicht offenbart. Zur Herstellung der Verbindungen der Formel I verestert man Biotin, d. h. die Verbindung der Formel

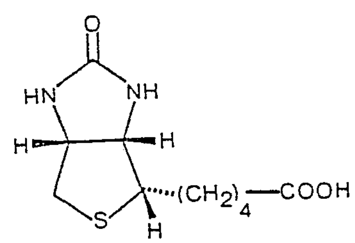

(II)

mit einem $C_{2-4}$-Alkanol.

Die Umsetzung kann unter an sich bekannten Veresterungsbedingungen durchgeführt werden. Die Veresterung wird zweckmässigerweise in Suspension im entsprechenden $C_{2-4}$-Alkohol, der als Reagens verwendet wird, durchgeführt. Zweckmässigerweise erfolgt die Veresterung in Gegenwart von sauren Katalysatoren, insbesondere von Säuren. Als geeignete Säuren für die Veresterung kommen z. B. Schwefelsäure, Salzsäure und Arylsulfonsäuren, wie p-Toluolsulfonsäure, in Betracht. Ein besonders bevorzugter saurer Katalysator ist die eben erwähnte p-Toluolsulfonsäure. Die Veresterung erfolgt zweckmässigerweise in einem Temperaturbereich zwischen Raumtemperatur und der Siedetemperatur des Reaktionsgemisches, vorzugsweise bei der letzterwähnten Temperatur.

Das erhaltene Produkt kann nach an sich bekannten Methoden isoliert und gereinigt werden.

Wie oben erwähnt, werden die Biotinester der Formel I durch die Haut besser aufgenommen als Biotin und dann durch Einwirkung des menschlichen Organismus in Biotin umgewandelt. Zudem sind die Ester im allgemeinen in wässrigen Alkoholen, also typischen Lösungs- bzw. Suspensionsmitteln für kosmetische Haarpflege- und Hautpflegemittel, leichter löslich als Biotin. Aus der nachstehenden Tabelle geht diese leichtere Löslichkeit hervor :

(Siehe Tabelle Seite 3f.)

Tabelle

Löslichkeiten (g/100 g) in wässrigem *Feinsprit

| Verbindung | Gewichtsverhältnis Feinsprit:Wasser | | |
|---|---|---|---|
| | 4:6 | 2:8 | 1:9 |
| Biotin | 0,15 | 0,04 | 0,03 |
| Biotinäthylester | >4 | 1,4 | 0,7 |
| Biotin-n-propylester | 1,9 | 0,5 | 0,4 |
| Biotin-n-butylester | 1,3 | 0,2 | 0,07 |
| Biotinisobutylester | 1,2 | 0,2 | 0,15 |

* 95 % Aethanol

In der Praxis deuten diese Ergebnis an, dass die Biotinester in kosmetischen Mitteln bei höheren Einsatzkonzentrationen verwendet werden können als Biotin.

Die Verbindungen der Formel I können zu verschiedenartigen kosmetischen Mitteln, insbesondere Haarpflege- und Hautpflegemitteln, formuliert werden. Das erfindungsgemässe kosmetische Haarpflege- oder Hautpflegemittel ist gekennzeichnet durch einen Gehalt an mindestens einer Verbindung der oben definierten allgemeinen Formel I. Neben dem Wirkstoff der Formel I können in der Kosmetik gebräuchliche Formulierungshilfsstoffe und eventuell andere kosmetisch wirksame Begleitstoffe vorliegen. Solche kosmetischen Haarpflege- bzw. Hautpflegemittel liegen z. B. in Form von Haarwässern, Shampoos, Frisiermitteln (Frisiergels), Haarsprays, Haarcrèmes und Hautcrèmes vor.

In den erfindungsgemässen kosmetischen Haarpflege- bzw. Hautpflegemitteln betragen die Konzentrationen an Wirkstoffen der Formel I im allgemeinen 0,01 bis 5,0 Gewichtsprozent, vorzugsweise 0,1 bis 0,5 Gewichtsprozent.

Als Formulierungshilfsstoffe bzw. kosmetisch wirksame Begleitstoffe für die erfindungsgemässen Haarpflege- und Hautpflegemittel kommen die üblichen, in der Kosmetik für solche Mittel verwendeten Zusätze in Betracht, z. B. Lösungs- oder Dispersionsmittel, wie Wasser und Alkohole, z. B. Aethanol ; kosmetische Grundkörper, wie solche mit auffettenden Eigenschaften ; Emulgatoren bzw. Lösungsvermittler ; Tenside als Netzmittel, Dispergatoren oder Waschrohstoffe ; Verdickungsmittel ; biozide Konservierungsmittel ; Stabilisatoren, wie UV-Absorber und Antioxidantien ; Parfüme ; farbgebende Mittel ; haut- bzw. haarnährende Mittel ; bakterientötende Mittel ; desodorierende Mittel ; Mittel zur Erhöhung des Hautmetabolismus oder der Hautelastizität ; und Mittel zur Behandlung von Dermatosen.

Liegen die erfindungsgemässen kosmetischen Haarpflege- bzw. Hautpflegemittel in Form von Sprays vor, so sind alle bei Aerosolgemischen herkömmlichen Treibgase, z. B. halogenierte Kohlenwasserstoffe, verwendbar.

Zur Herstellung der erfindungsgemässen kosmetischen Haarpflege- und Hautpflegemittel wird mindestens ein Wirkstoff der oben definierten allgemeinen Formel I mit in der Kosmetik gebräuchlichen Formulierungshilfsstoffen und eventuell mit einem oder mehreren anderen kosmetisch wirksamen Begleitstoffen vermischt. Das Vermischen kann einstufig oder mehrstufig und auf in der Kosmetik übliche Weise erfolgen.

Die erfindungsgemässen kosmetischen Haarpflege- bzw. Hautpflegemittel werden verwendet, indem man auf die zu behandelnde Kopfhaut bzw. Haut eine wirksame Menge des erfindungsgemässen kosmetischen Haarpflege bzw. Hautpflegemittels appliziert. Diese Applikation kann auf in der Kosmetik übliche Weise erfolgen, z. B. durch Einreiben oder Besprühen.

Die nachfolgenden Beispiele dienen zur näheren Erläuterung der Erfindung.

I. Herstellung der Wirkstoffe der Formel I

Beispiel 1

100 g Biotin werden in 2 l absolutes Aethanol eingetragen und unter Rühren mit einer Lösung von 12,2 g p-Toluolsulfonsäure in Aethanol versetzt. Danach erhitzt man das Gemisch während 4 Stunden unter Rühren bei Rückflusstemperatur, kühlt die resultierende Lösung in einem Eisbad ab und filtriert den weissen Niederschlag ab.

Beim Einengen der Lösung und anschliessenden Kühlen bildet sich eine weitere Menge Niederschlag. Die vereinigten weissen Niederschläge werden dann in Wasser suspendiert, mit Natronlauge bis zu einem pH-Wert von 7,0 versetzt und schliesslich abfiltiert. Das Reaktionsprodukt wird bei 40 °C im Vakuum getrocknet. Man erhält Biotinäthylester als weisses Pulver, Smp. 124-125°C ; Ausbeute 80 g.

Mikroanalyse ($C_{12}H_{20}N_2O_3S$) :
berechnet : C 52,92 H 7,40 N 10,29 S 11,77 %
gefunden (wasserfrei) : C 52,75 H 7,68 N 10,27 S 11,82 %

Auf analoge Weise werden die folgenden Biotin-ester hergestellt :
Biotin-n-propylester, Smp. 124 °C
Biotin-n-butylester, Smp. 120-121 °C
Biotinisobutylester, Smp. 124 °C.

II. Formulierungsbeispiele

Beispiel 2

Ein Haarwasser hat die folgende Zusammensetzung :

| | |
|---|---|
| Wirkstoff der Formel I, z. B. Biotinäthylester | 0,50 g |
| Uvinul® D 50 (2, 2', 4, 4'-Tetrahydroxybenzophenon) (1) | 0,05 g |
| Diisopropyladipat | 0,20 g |
| Cremophor® RH 60 (äthoxyliertes hydriertes Ricinusöl) (1) | 0,10 g |
| Parfüm | 0,10 g |
| Feinsprit (95 % Aethanol) | 45,00 g |
| D-Panthenol-äthyläther | 0,15 g |
| Farbstoff | (Menge nach Bedarf) |
| Entmineralisiertes Wasser | ad 100,00 g |

Methode

In einem mit Propellerrührer versehenen Herstellungskessel aus rostfreiem Stahl wird der Feinsprit vorgelegt. Danach werden der Wirkstoff, das Uvinul® D 50, das Diisopropyladipat, das Cremophor® RH 60 und das Parfüm zugefügt und unter kräftigem Rühren in Lösung gebracht. Schliesslich wird eine Lösung des D-Panthenol-äthyläthers und eventuell auch des Farbstoffes im entmineralisierten Wasser zugegeben und das Ganze homogen verrührt. Auf diese Weise wird eine klare, eventuell gefärbte Lösung erhalten, die sich als Haarwasser eignet.

Beispiel 3

Ein klares Shampoo hat die folgende Zusammensetzung :

| | |
|---|---|
| Wirkstoff der Formel I, z. B. Biotinäthylester | 0,50 g |
| Texapon® ASV (Gemisch von Fettalkoholäthersulfaten) (2) | 25,00 g |
| Phenonip® (Gemisch von p-Hydroxybenzoesäureestern, gelöst in 2-Phenoxyäthanol) (3) | 0,50 g |
| D-Panthenol-äthyläther | 0,50 g |
| D-Panthenol | 0,50 g |
| 1,2-Propylenglykol | 1,00 g |
| Cetiol® HE (Polyolpolyäther-Fettsäureester) (2) | 1,00 g |
| Tego®-Betain L 7 (N-(3-Acylamidopropyl)-N,N-dimethylammonioacetate) (4) | 20,00 g |
| Natriumchlorid (rein) | 0,50 g |
| Parfüm | (Menge nach Bedarf) |
| Farbstoff | (Menge nach Bedarf) |
| Entmineralisiertes Wasser | ad 100,00 g |

Methode

In einem mit Rührwerk, Abstreifern, Gegenrührwerk und Thermometer versehenen, heizbaren Kessel aus rostfreiem Stahl werden das Texapon® ASV, das Phenonip®, eventuell das Parfüm sowie ein Viertel der benötigten Menge Wasser vorgelegt und unter langsamen Rühren auf 45 °C erwärmt. Anschliessend wird

der Wirkstoff beigefügt und unter Rühren bei 45 °C in Lösung gebracht und danach die Heizung abgestellt.

Der D-Panthenol-äthyläther, das D-Panthenol, das 1,2-Propylenglykol, das Cetiol® HE und eventuell Farbstoff werden zusammen in ca. 85 % der restlichen Menge Wasser unter Rühren gelöst. Danach wird das Tego®-Betain L 7 zugefügt und das Ganze homogen verrührt. Die erhaltene Lösung wird dem Ansatz im Kessel beigefügt.

Das Natriumchlorid wird in der restlichen Menge Wasser gelöst und die sich ergebende, ca. 10-prozentige Lösung dem Ansatz im Kessel zugegeben. Der Kesselinhalt wird dann homogen verrührt und auf Raumtemperatur abgekühlt. Ein klares, ggf. gefärbtes und/oder parfümiertes Shampoo wird erhalten.

## Beispiel 4

Eine Oel/Wasser-Crème (Hautcrème) hat die folgende Zusammensetzung :

| | |
|---|---|
| Wirkstoff der Formel I, z. B. Biotinäthylester | 0,50 g |
| Tagat® S (Polyoxyäthylenglycerinmonostearat) (4) | 3,00 g |
| Glycerinmonostearat | 2,00 g |
| Stearylalkohol | 1,00 g |
| Aethyladipat | 8,00 g |
| PCL flüssig (Gemisch alkylverzweigter Fettsäureester) (5) | 2,00 g |
| Deltyl-Extra® (Isopropylmyristat) (6) | 3,00 g |
| Cetiol® HE (Polyolpolyäther-Fettsäureester) (2) | 2,00 g |
| Aquaphil® K (Gemisch von Lanolin und Lanolin-derivaten) (7) | 1,00 g |
| Carbopol® 940 (Polyacrylsäure) (8) | 0,40 g |
| Natriumhydroxid (rein) | 0,04 g |
| D-Panthenol-äthyläther | 0,50 g |
| D-Panthenol | 2,00 g |
| Phenonip® (Gemisch von p-Hydroxybenzoesäureestern, gelöst in 2-Phenoxyäthanol) (3) | 0,80 g |
| 1,2-Propylenglykol | 5,00 g |
| Parfüm | (Menge nach Bedarf) |
| Entmineralisiertes Wasser | ad 100,00 g |

Methode

(i) Herstellung der Wasserphase

In einem mit Ankerrührer, Abstreifern, zweitem gegenläufigem Rührwerk, Thermometer und eingebautem Homogenisator versehenen, heiz- und kühlbaren Kessel aus rostfreiem Stahl werden bei Raumtemperatur ca. 90 % der benötigten Menge Wasser, das 1,2-Propylenglykol und das Phenonip® vorgelegt. Das Carbopol® 940 wird aufgestreut und unter Rühren dispergiert. Man lässt den Kesselgehalt 16 Stunden quellen, danach auf 80 °C aufheizen und solange homogenisieren, bis sämtliche eventuell noch vorhandenen Carbopol®-Klumpen verschwunden sind. Anschliessend wird eine ca. 10-prozentige wässrige Lösung des Natriumhydroxids zugegeben und das Ganze bei 80 °C homogen verrührt. Auf diese Weise wird die Wasserphase erhalten.

(ii) Herstellung der Fettphase

In einem mit Ankerrührer, Abstreifern und Thermometer ausgestatteten Schmelzkessel aus rostfreiem Stahl wird ein Gemisch von Tagat® S, Glycerinmonostearat, Stearylalkohol, Aethyladipat, PCL flüssig, Deltyl-Extra®, Cetiol® HE, Aquaphil® K und dem Wirkstoff auf 80 °C erwärmt und unter leichtem Rühren geschmolzen. Es ergibt sich somit eine klare Schmelze (die Fettphase).

(iii) Fertigstellung der Crème

Die Fettphase wird der Wasserphase zugefügt, und anschliessend wird das Gemisch ca. 10 Minuten homogenisiert und 30 bis 60 Minuten bei 80 °C weitergerührt. Danach wird innert ca. 1 Stunde auf Raumtemperatur abgekühlt, währenddessen bei 45 °C der D-Panthenol-äthyläther, das D-Panthenol und ggf. das Parfüm, gelöst in der restlichen Menge Wasser, zugegeben werden. Es ergibt sich dabei eine weisse homogene Emulsion, die sich als Hautcrème eignet.

Hersteller der Bestandteile (1)-(8) :

(1) BASF, 6 700 Ludwigshafen/Rhein, BRD
(2) Henkel KGaA, Düsseldorf, BRD
(3) Nipa Laboratories Ltd., Industrial Estate, Treforest, South Wales, GB

(4) Th. Goldschmidt AG, D-4300 Essen 1, BRD
(5) Dragoco GmbH, D-3450 Holzminden, BRD
(6) Givaudan & Cie, CH-1214 Vernier-Genève, Schweiz
(7) Westbrook Lanolin Company, Argonaut Works, Laisterdyke, Bradford, Yorkshire, GB
(8) B.F. Goodrich Chemical Corporation, 3135 Euclid Avenue, Cleveland, Ohio 44115, USA

**Patentansprüche** (für die Vertragsstaaten : BE, CH, DE, FR, GB, IT, LI, NL, SE)

1. Verbindungen der allgemeinen Formel

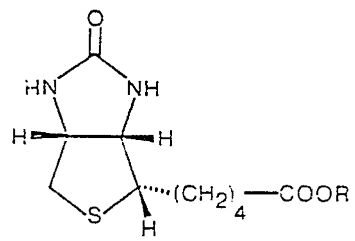

(I)

wobei R $C_{2-4}$-Alkyl bedeutet, zur topischen Anwendung in Verfahren zur therapeutischen Behandlung.

2. Kosmetisches Haarpflege- oder Hautpflegemittel, gekennzeichnet durch einen Gehalt an mindestens einer Verbindung nach Anspruch 1.

3. Kosmetisches Haarpflege- oder Hautpflegemittel nach Anspruch 2, gekennzeichnet durch einen Gehalt von Biotinäthylester.

4. Kosmetisches Mittel nach Anspruch 2 oder 3 dadurch gekennzeichnet, daß es in Form eines Haarwassers, eines Shampoos oder einer Hautcrème vorliegt.

5. Verwendung von Verbindungen der allgemeinen Formel

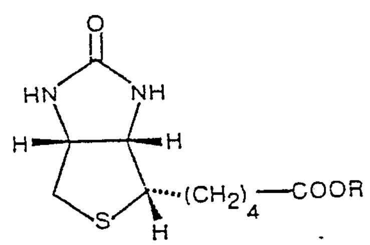

(I)

wobei R $C_{2-4}$-Alkyl bedeutet, zur Herstellung von Haarpflege- oder Hautpflegemitteln.

**Patentansprüche** (für den Vertragsstaat AT)

1. Kosmetisches Haarpflege- oder Hautpflegemittel, dadurch gekennzeichnet, dass es eine wirksame Menge mindestens einer Verbindung der allgemeinen Formel

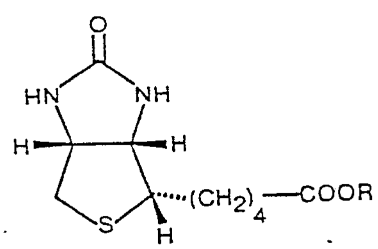

(I)

worin R $C_{2-4}$-Alkyl bedeutet, sowie in der Kosmetik gebräuchliche Formulierungshilfsstoffe enthält.

2. Kosmetisches Haarpflege- oder Hautpflegemittel nach Anspruch 1, dadurch gekennzeichnet, dass es als Verbindung der Formel I Biotinäthylester enthält.

3. Kosmetisches Haarpflege- oder Hautpflegemittel nach Anspruch 1, dadurch gekennzeichnet, dass es als Verbindung(en) der Formel I mindestens eine Verbindung ausgewählt aus Biotin-n-propylester, Biotin-n-butylester und Biotinisobutylester enthält.

4. Kosmetisches Mittel nach einem der Ansprüche 1 bis 3, dadurch gekennzeichnet, dass es in Form eines Haarwassers, eines Shampoos oder einer Hautcrème vorliegt.

**Claims** (for the Contracting States : BE, CH, DE, FR, GB, IT, LI, NL, SE)

1. Compounds of the general formula

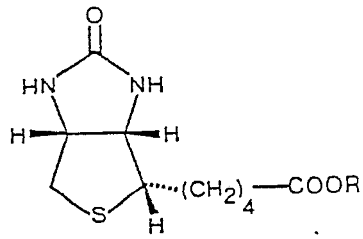

(I)

wherein R is $C_{2-4}$ alkyl, for topical use in therapeutic methods.

2. Cosmetic hair care or skin care composition, characterised by a content of at least one compound according to claim 1.

3. Cosmetic hair care or skin care composition according to claim 2, characterised by a content of biotin ethyl ester.

4. Cosmetic composition according to claim 2 or claim 3, characterised in that it is in the form of a hair wash, shampoo or skin cream.

5. The use of compounds of the general formula

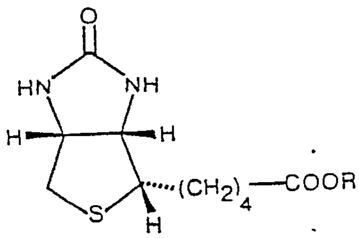

(I)

wherein R is $C_{2-4}$ alkyl, for the preparation of hair care or skin care compositions.

**Claims** (for the Contracting State AT)

1. Cosmetic hair care or skin care composition, characterised in that it contains an effective amount of at least one compound of the general formula

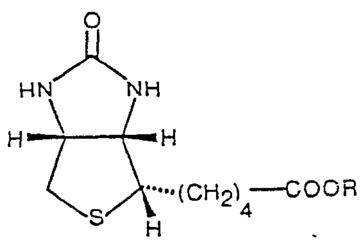

(I)

wherein R is $C_{2-4}$ alkyl, as well as formulation components conventional in cosmetics.

2. Cosmetic hair care or skin care composition according to claim 1, characterised in that it contains, as the compound of formula I, biotin ethyl ester.

3. Cosmetic hair care or skin care composition according to claim 1, characterised in that it contains, as compound(s) of formula I, at least one compound selected from biotin n-propyl ester, biotin n-butyl ester and biotin isobutyl ester.

4. Cosmetic composition according to one of claims 1 to 3, characterised in that it is in the form of a hair wash, shampoo or skin cream.

**Revendications** (pour les Etats contractants : BE, CH, DE, FR, GB, IT, LI, NL, SE)

1. Composés de formule générale :

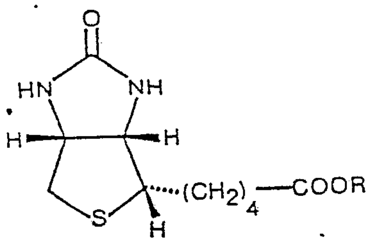

(I)

dans laquelle R représente un groupe alkyle en $C_2$ à $C_4$, pour application topique dans un procédé de traitement thérapeutique.

2. Produit cosmétique pour soins capillaires ou soins de la peau, caractérisé en ce qu'il contient au moins un composé selon la revendication 1.

3. Produit cosmétique pour soins capillaires ou soins de la peau selon la revendication 2, caractérisé en ce qu'il contient l'ester éthylique de la biotine.

4. Produit cosmétique selon la revendication 2 ou 3, caractérisé en ce qu'il se présente sous forme d'une eau capillaire, d'un shampooing ou d'une crème pour la peau.

5. Utilisation de composés de formule générale :

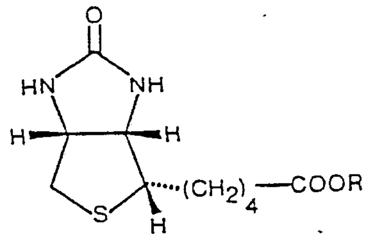

(I)

dans laquelle R représente un groupe alkyle en $C_2$ à $C_4$, pour préparer des produits capillaires ou pour soins de la peau.

**Revendications** (pour l'Etat contractant AT)

1. Produit cosmétique pour soins capillaires ou soins de la peau, caractérisé en ce qu'il contient une quantité active d'au moins un composé de formule générale

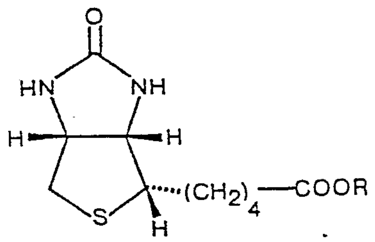

(I)

dans laquelle R représente un groupe alkyle en $C_2$ à $C_4$, ainsi que les adjuvants de formulation usuels en cosmétiques.

2. Produit cosmétique pour soins capillaires ou soins de la peau selon la revendication 1, caractérisé en ce qu'il contient l'ester éthylique de la biotine à titre de composé de formule I.

3. Produit cosmétique pour soins capillaires ou soins de la peau selon la revendication 1, caractérisé en ce qu'il contient, à titre de composé(s) de formule I, au moins un composé choisi parmi l'ester de n-propyle de la biotine, l'ester de n-butyle de la biotine, et l'ester d'isobutyle de la biotine.

4. Produit cosmétique selon l'une des revendications 1 à 3, caractérisé en ce qu'il se présente sous forme d'une eau capillaire, d'un shampooing ou d'une crème pour la peau.